(19) Europäisches Patentamt

European Patent Office

Office européen des brevets

(11) EP 0 609 750 B1

(12) **EUROPÄISCHE PATENTSCHRIFT**

(45) Veröffentlichungstag und Bekanntmachung des Hinweises auf die Patenterteilung:
16.10.1996 Patentblatt 1996/42

(51) Int. Cl.$^6$: **B01J 23/88**

(21) Anmeldenummer: 94101021.7

(22) Anmeldetag: 25.01.1994

(54) **Multimetalloxidmassen**

Multimetal oxide masses

Masses d'oxydes multimétalliques

(84) Benannte Vertragsstaaten:
**BE DE ES FR GB IT NL**

(30) Priorität: **03.02.1993 DE 4302991**

(43) Veröffentlichungstag der Anmeldung:
**10.08.1994 Patentblatt 1994/32**

(73) Patentinhaber: **BASF Aktiengesellschaft**
**67063 Ludwigshafen (DE)**

(72) Erfinder:
• **Tenten, Andreas, Dr.**
**D-67434 Neustadt (DE)**
• **Weidlich, Peter, Dr.**
**D-68159 Mannheim (DE)**

(56) Entgegenhaltungen:
**EP-A- 0 017 000**          **EP-A- 0 052 839**
**EP-A- 0 235 760**          **US-A- 4 289 654**
**US-A- 4 358 608**          **US-A- 4 380 672**

**Beschreibung**

Die vorliegende Erfindung betrifft Multimetalloxidmassen, die als Grundbestandteile die Elemente Mo, V, W, Cu und Ni in oxidischer Form mit der Maßgabe enthalten, daß zwischen den verschiedenen elementaren Konstituenten die nachfolgenden molaren Verhältnisse vorliegen:

Mo:V = 12:1 bis 2:1,
Mo:W = 60:1 bis 3:1,
Mo:Cu = 24:1 bis 2:1 und
Cu:Ni = 5:1 bis 1:3.

Außerdem betrifft die vorliegende Erfindung ein Verfahren zur Herstellung dieser Massen, sowie deren Verwendung.

Die US-A-4 289 654, die EP-A 427 508, die DE-A 29 09 671, die DE-C 31 51 805 und die DE-AS 26 26 887 offenbaren Multimetalloxidmassen, die neben anderen Elementoxiden als Grundbestandteile die Elemente Mo, V, W und Cu in oxidischer Form enthalten. Beispielhaft offenbart werden u.a. solche Multimetalloxidmassen, in denen die molaren Konstituentenverhältnisse Mo:V, Mo:W und Mo:Cu im erfindungsgemäß definierten Bereich liegen. Diese Multimetalloxidmassen werden u.a. als Katalysatoren für die katalytische Gasphasenoxidation von Acrolein zu Acrylsäure empfohlen.

Hinsichtlich dieses Verwendungszwecks vermögen diese Multimetalloxidmassen jedoch in Bezug auf Aktivität und Selektivität der Acrylsäurebildung nicht voll zu befriedigen. Ferner weisen diese Multimetalloxidmassen bei dieser Verwendung ein ausgeprägtes Formierungsverhalten auf. D.h. bei Verwendung von frisch hergestellten Mulitmetalloxidmassen wächst die Selektivität (bei vorgegebenem Umsatz) der Acrylsäurebildung erst mit zunehmender Betriebsdauer auf ihren Endwert.

Die EP-A 235 760 offenbart als Katalysatoren zur gasphasenkatalytischen Oxidation von Acrolein zu Acrylsäure geeignete Multimetalloxidmassen, die neben den Elementen Mo, V, W und Cu als weiterer Grundbestandteil Ni in oxidischer Form enthalten. Nachteilig an diesen Multimetalloxidmassen ist jedoch, daß in ihnen das molare Verhältnis des elementaren Konstituenten Cu zum elementaren Konstituenten Ni unterhalb von 1:3 liegt, weshalb auch diese Multimetalloxidmassen bezüglich ihrer Verwendung zur katalytischen Gasphasenoxidation von Acrolein zu Acrylsäure nicht voll zu befriedigen vermögen.

Aufgabe der vorliegenden Erfindung war daher, Multimetalloxidmassen zur Verfügung zu stellen, die bei Verwendung als Katalysatoren für die gasphasenkatalytische Oxidation von Acrolein zu Acrylsäure besser zu befriedigen vermögen. Demgemäß wurden die eingangs definierten Massen gefunden.

Die Aufgabe wird durch das Produkt gemäß Anspruch 1 gelöst.

Die Verwendung des Produkts sowie das Verfahren zu seiner Herstellung werden in den Ansprüchen 3, 4 und 6 beansprucht. Bevorzugte Ausführungsformen des Produkts sind in dem Ansprüchen 2 und 5 beansprucht.

Bevorzugt sind solche erfindungsgemäßen Multimetalloxidmassen, in denen das molare Verhältnis des elementaren Konstituenten Cu zum elementaren Konstituenten Ni 3:1 bis 1:2, besonders bevorzugt 2:1, beträgt.

Besonders vorteilhafte erfindungsgemäße Massen sind dabei solche, die der allgemeinen Summenformel I

$$Mo_{12}V_aW_bCu_cNi_dX^1_eX^2_fX^3_gX^4_hX^5_iO_n \qquad (I),$$

in der die Variablen folgende Bedeutung haben:

$X^1$     ein oder mehrere Alkalimetalle,
$X^2$     ein oder mehrere Erdalkalimetalle,
$X^3$     Chrom, Mangan, Cer und/oder Niob,
$X^4$     Antimon und/oder Wismut,
$X^5$     Silicium, Aluminium, Titan und/oder Zirkonium,
a     1 bis 6,
b     0,2 bis 4,

c     0,5 bis 6
d     0,2 bis 6    } und c:d = 5:1 bis 1:3,

e     0 bis 2,
f     0 bis 3,
g     0 bis 5,

h      0 bis 40,

i      0 bis 40 und

n      eine Zahl, die durch die Wertigkeit und Häufigkeit der von Sauerstoff verschiedenen Elemente in I bestimmt wird,

entsprechen.

Unter diesen sind wiederum jene bevorzugt, bei denen die stöchiometrischen Koeffizienten e, f, g und h gleich 0 sind.

Die Herstellung der erfindungsgemäßen Massen erfolgt in an sich bekannter Weise in der Regel so, daß man aus geeigneten Ausgangsverbindungen, die die elementaren Katalysatorkonstituenten als Bestandteil enthalten, ein möglichst inniges Trockengemisch herstellt und dieses bei Temperaturen von 200 bis 500°C, vorzugsweise 300 bis 400°C, calciniert. Wesentlich ist nur, daß es sich bei den eingesetzten Ausgangsverbindungen entweder bereits um Oxide handelt, oder um solche Verbindungen, die durch Erhitzen, gegebenenfalls in Anwesenheit von Sauerstoff, in Oxide überführbar sind. Neben den Metalloxiden kommen daher als Ausgangsverbindungen vor allem Halogenide, Nitrate, Formiate, Acetate, Carbonate oder Hydroxide in Betracht. Geeignete Ausgangsverbindungen des Mo, W und V sind auch deren Molybdate, Wolframate und Vanadate bzw. die von diesen abgeleiteten Säuren.

Das innige Vermischen der Ausgangsverbindungen kann in trockener oder in nasser Form erfolgen. Erfolgt es in trockener Form, verden die Ausgangsverbindungen zweckmäßigerweise als feinteilige Pulver eingesetzt und nach dem Mischen z.B. zu Katalysatorkörpern gewünschter Geometrie verpreßt, (z.B. tablettiert), die dann der Calcinierung unterworfen werden.

Vorzugsweise erfolgt das innige Vermischen jedoch in nasser Form. Üblicherweise werden die Ausgangsverbindungen dabei in Form einer wäßrigen Losung oder Suspension miteinander vermischt. Anschließend wird die wäßrige Masse getrocknet und nach Trocknung calciniert.

Vorzugsweise erfolgt der Trocknungsprozeß unmittelbar im Anschluß an die Fertigstellung der wäßrigen Mischung und durch Sprühtrocknung (die Ausgangstemperaturen betragen in der Regel 100 bis 150°C). Das dabei anfallende Pulver kann unmittelbar durch Pressen geformt werden. Häufig erweist es sich jedoch für eine unmittelbare Weiterverarbeitung als zu feinteilig, weshalb es unter Zusatz von Wasser zweckmäßigerweise zunächst geknetet wird.

Die anfallende Knetmasse wird anschließend entweder zur gewünschten Katalysatorgeometrie geformt, getrocknet und dann der Calcinierung unterworfen (führt zu sogenannten Vollkatalysatoren) oder ungeformt calciniert und danach zu einem feinteiligen Pulver (üblicherweise < 80 $\mu$m) vermahlen, welches normalerweise unter Zusatz einer geringen Menge Wasser sowie gegebenenfalls weiterer üblicher Bindemittel als Feuchtmasse auf inerte Trägerkörper aufgetragen wird. Nach beendeter Beschichtung wird nochmals getrocknet und so der einsatzfähige Schalenkatalysator erhalten. Prinzipiell kann das calcinierte Pulver aber auch als Pulverkatalysator eingesetzt werden.

Im Rahmen der wäßrigen Naßvermischung der Ausgangsverbindungen werden als Ausgangsverbindungen der Konstituenten Cu und Ni vorzugsweise in Wasser lösliche Kupfer- und Nickelsalze wie Nitrate oder Acetate eingesetzt, wobei die Verwendung der Acetate von besonderem Vorteil ist. Selbstverständlich können die Acetate auch im Rahmen des Vermischens in situ gebildet werden (z.B. durch Verwendung von Carbonaten als Ausgangsverbindungen und Zusatz von Essigsäure).

Im Fall von Vollkatalysatoren beträgt der stöchiometrische Koeffizient i in der allgemeinen Summenformel I mit Vorteil 15 bis 40. Ansonsten weist i vorzugsweise den Wert 0 auf, da es sich bei den Oxiden des Konstituenten $X^5$ um solche handelt, die im wesentlichen lediglich eine Verdünnung der Restmasse bedingen.

Erfolgt das Vermischen der Ausgangsverbindungen in Form einer wäßrigen Lösung, so können mit selbiger auch inerte poröse Trägerkörper getränkt, getrocknet und abschließend zu Trägerkatalysatoren calciniert werden. Vollkatalysatoren weisen vorzugsweise eine Hohlzylindern entsprechende Geometrie gemäß der DE-A 31 13 179 auf.

Bei Verwendung der erfindungsgemäßen Multimetalloxidmassen als Katalysatoren für die gasphasenkatalytische Oxidation von Acrolein zu Acrylsäure erfolgt deren Einsatz jedoch vorzugsweise in Form von Schalenkatalysatoren. Dabei können die üblichen Trägermaterialien wie poröse oder unporöse Aluminiumoxide, Siliciumdioxid, Thoriumdioxid, Zirkondioxid, Siliciumcarbid oder Silicate wie Magnesium- oder Aluminiumsilicat verwendet werden. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Unter diesen sind wiederum Kugeln besonders vorteilhaft. Ganz besonders vorteilhaft ist die Verwendung von im wesentlichen unporösen, oberflächenrauhen kugelförmigen Trägern aus Steatit, deren Durchmesser 1 bis 6 mm, bevorzugt 4 bis 5 mm beträgt. Die Schichtdicke der Aktivmasse wird zweckmäßigerweise als im Bereich 50 bis 500 $\mu$m, bevorzugt im Bereich 150 bis 250 $\mu$m liegend, gewählt. Es sei an dieser Stelle darauf hingewiesen, daß bei der Herstellung von Schalenkatalysatoren das Beschichten der Trägerkörper auch vor der Calcinierung, d.h. z.B. mit dem befeuchteten Sprühpulver, erfolgen kann.

Die Calcinierung der die Ausgangsverbindungen in inniger Mischung enthaltenden Trochenmasse erfolgt ganz generell mit Vorteil in einem mit Luft beschickten Drehrohrofen.

Die Beschichtung der Trägerkörper wird zur Herstellung der Schalenkatalysatoren in der Regel in einem geeigneten drehbaren Behälter ausgeführt, wie er z.B. aus der DE-A 29 09 671 oder aus der EP-A 293 859 vorbekannt ist.

3

Mit Vorteil werden die Ausgangsverbindungen, das Beschichtungsverfahren sowie die Calcinierungsbedingungen gemäß der EP-A 293 859 in an sich bekannter Weise so gewählt, daß die resultierenden Multimetalloxidaktivmassen eine spezifische Oberfläche von 0,50 bis 150 $m^2$/g, ein spezifisches Porenvolumen von 0,10 bis 0,90 $cm^3$/g und eine solche Porendurchmesser-Verteilung aufweisen, daß auf die Durchmesserbereiche 0,1 bis < 1 $\mu$m, 1,0 bis < 10 $\mu$m und 10 $\mu$m bis 100 $\mu$m jeweils wenigstens 10 % des Porengesamtvolumens entfallen. Vorzugsweise werden die in der EP-A 293 859 als bevorzugt genannten Porendurchmesser-Verteilungen eingestellt.

Die erfindungsgemäßen Multimetalloxidmassen eignen sich insbesondere als Katalysatoren mit erhöhter Aktivität und Selektivität für die gasphasenkatalytische Oxidation von Acrolein zu Acrylsäure. Als Acrolein wird dabei vorzugsweise das Reaktionsprodukt einer entsprechenden katalytischen Gasphasenoxidation des Propens eingesetzt. Üblicherweise wird die gasphasenkatalytische Oxidation des Acroleins in Rohrbündelreaktoren als heterogene Festbettoxidation ausgeführt. Als Oxidationsmittel wird in an sich bekannter Weise Sauerstoff, zweckmäßigerweise mit inerten Gasen verdünnt, eingesetzt. Geeignete Inertgase sind z.B. $N_2$ und/oder Wasserdampf. Reaktionstemperatur und Reaktionsdruck sind dem Fachmann bekannt (vgl. DE-A 41 32 263). Bemerkenswerterweise weisen die erfindungsgemäßen Multimetalloxidkatalysatoren bezüglich der Selektivität der Acrylsäurebildung auch eine reduzierte Formierungszeit auf; d.h., wird ein mit den erfindungsgemäßen Massen beschickter Rohrbündelreaktor unter dem Fachmann an sich bekannten Bedingungen mit einem Acrolein enthaltenden Gasstrom zum Zwecke der oxidativen Bildung von Acrylsäure betrieben, so wächst die Selektivität der Acrylsäurebildung bereits innerhalb einer reduzierten Betriebsdauer auf den maximalen Plateauwert an.

Die erfindungsgemäßen Multimetalloxidmassen eignen sich aber auch zur gasphasenkatalytischen Oxidation anderer organischer Verbindungen wie Alkane, Alkanole, Alkanale, Alkene und anderer Alkenale zu olefinisch ungesättigten Aldeyhden und/oder Carbonsäuren, sowie den entsprechenden Nitrilen (Ammonoxidation, z.B. Propen zu Acrylnitril und 2-Methylpropen bzw. tert.-Butanol zu Methacrylnitril). Die Anzahl der C-Atome besagter organischer Verbindungen liegt normalerweise im Bereich von 3 bis 6 C-Atomen, vorzugsweise beträgt die Anzahl der C-Atome 3 oder 4.

Beispiele

a) Allgemeine Herstellvorschrift für Multimetalloxidmassen

Als Vergleichsbeispiel wurde eine katalytisch aktive Masse V1 der Zusammensetzung $Mo_{12}V_3W_{1,2}Cu_{2,4}O_X$ wie folgt hergestellt:

190 g Kupfer(II)acetatmonohydrat wurden in 2.700 g Wasser zu einer Lösung I gelöst. In 5.500 g Wasser wurden bei 95°C nacheinander 860 g Ammoniumheptamolybdattetrahydrat, 143 g Ammoniummetavanadat und 126 g Ammoniumparawolframatheptahydrat zu einer Lösung II gelöst. Anschließend wurde die Lösung I auf einmal in die Lösung II eingerührt und das wäßrige Gemisch bei einer Austrittstemperatur von 110°C sprühgetrocknet. Danach wurde das Sprühpulver je kg Pulver mit 0,15 kg Wasser verknetet. Die Knetmasse wurde in einem mit Luft beschickten Drehrohrofen innerhalb von 3 h auf 400°C aufgeheizt und anschließend 5 h bei 400°C calciniert. Das calcinierte, katalytisch aktive Material wurde auf Teilchendurchmesser von 0,1 bis 50 $\mu$m gemahlen.

In völlig entsprechender Weise wurden Multimetalloxidpulver der nachfolgenden Zusammensetzungen hergestellt (Ni wird als Nickel(II)acetattetrahydrat eingesetzt):

$$Mo_{12}V_3W_{1,2}Cu_{1,6}Ni_{0,8}O_X \qquad (B1)$$

$$Mo_{12}V_3W_{1,2}Cu_{0,8}Ni_{1,6}O_X \qquad (B2)$$

$$Mo_{12}V_3W_{1,2}Ni_{2,4}O_X \qquad (V2)$$

$$Mo_{12}V_3W_{1,2}Cu_{2,0}Ni_{0,4}O_X \qquad (B3)$$

$$Mo_{12}V_3W_{1,2}Cu_{2,4}Ni_{0,8}O_{X'} \qquad (B4)$$

$$Mo_{12}V_3W_{1,2}Cu_{1,6}O_{X''} \qquad (V3)$$

$$Mo_{12}V_3W_{1,2}Cu_{0,4}Ni_{2,0}O_X \qquad (V4)$$

b) Herstellung von Schalenkatalysatoren

Mit den in a) erhaltenen Aktivmassenpulvern wurden in einer Drehtrommel unporöse, oberflächenrauhe Steatitkugeln eines Durchmessers von 4 bis 5 mm in einer Menge von 50 g Pulver je 200 g Steatitkugeln bei gleichzeitigem Zusatz von 18 g Wasser beschichtet. Anschließend wurde mit 110°C heißer Luft getrocknet.

c) Gasphasenoxidation von Acrolein

Ein Reaktionsrohr (V2A, 2 mm Wandstärke) eines freien Durchmessers von 25 mm wurde jeweils mit 1 l eines Gemisches bestehend aus den in b) erhaltenen Schalenkatalysatoren und diese verdünnendes Inertmaterial befüllt und mit einem Salzbad beheizt. Dann wurde es mit 2.300 Nl/h einer Gasmischung der Zusammensetzung 5 Vol.-% Acrolein, 7 Vol.-% Sauerstoff, 10 Vol.-% Wasserdampf und als Restmenge Stickstoff, beschickt.

Die Salzbadtemperatur wurde in allen Fällen so eingestellt, daß bei einfachem Durchgang eine Acroleinumwandlung von ca. 99 mol-% resultierte. Die diesbezüglich als Funktion des jeweils eingesetzten Schalenkatalysators nach dreiwöchiger Betriebsdauer erforderlichen Salzbadtemperaturen (Maß für die Aktivität; geringere Salzbadtemperaturen weisen erhöhte Aktivitäten aus) sowie die resultierenden Selektivitäten (bezogen auf gebildete Acrylsäure) zeigt die Tabelle. Sie enthält ebenfalls die bezüglich der Acrylsäureselektivität bestimmten Formierungszeiten.

Tabelle

| Katalysator | Acroleinumwandlung [mol-%] | Salzbadtemperatur [°C] | Selektivität [mol-%] | Formierungszeit [Tage] |
|---|---|---|---|---|
| V1 | 99,1 | 275 | 95,0 | 10 |
| B1 | 99,0 | 264 | 96,2 | 2 |
| B2 | 98,9 | 265 | 95,8 | 3 |
| V2 | 98,2 | 283 | 94,3 | 3 |
| B3 | 99,1 | 267 | 95,7 | 3 |
| B4 | 98,9 | 265 | 95,6 | 4 |
| V3 | 99,0 | 267 | 95,3 | 8 |
| V4 | 98,5 | 279 | 94,5 | 3 |

**Patentansprüche**

1. Multimetalloxidmassen, die als Grundbestandteile die Elemente Mo, V, W, Cu und Ni in oxidischer Form mit der Maßgabe enthalten, daß zwischen den verschiedenen elementaren Konstituenten die nachfolgenden molaren Verhältnisse vorliegen:

   Mo:V = 12:1 bis 2:1,
   Mo:W = 60:1 bis 3:1
   Mo:Cu = 24:1 bis 2:1 und
   Cu:Ni = 5:1 bis 1:3.

2. Multimetalloxidmassen nach Anspruch 1, die der allgemeinen Summenformel I

$$Mo_{12}V_aW_bCu_cNi_dX^1_eX^2_fX^3_gX^4_hX^5_iO_n \qquad (I),$$

in der die Variablen folgende Bedeutung haben:

$X^1$      ein oder mehrere Alkalimetalle,
$X^2$      ein oder mehrere Erdalkalimetalle,
$X^3$      Chrom, Mangan, Cer und/oder Niob,
$X^4$      Antimon und/oder Wismut,
$X^5$      Silicium, Aluminium, Titan und/oder Zirkonium,
a      1 bis 6,

b 0,2 bis 4,

$$c \quad 0,5 \text{ bis } 6 \left.\right\} \quad \text{und } c:d = 5:1 \text{ bis } 1:3,$$
$$d \quad 0,2 \text{ bis } 6$$

e 0 bis 2,
f 0 bis 3,
g 0 bis 5,
h 0 bis 40,
i 0 bis 40 und
n eine Zahl, die durch die Wertigkeit und Häufigkeit der von Sauerstoff verschiedenen Elemente in I bestimmt wird,

entsprechen.

3. Verwendung von Multimetalloxidmassen gemäß Anspruch 1 als Katalysator zur gasphasenkatalytischen Oxidation von Acrolein zu Acrylsäure.

4. Verfahren zur Herstellung von Multimetalloxidmassen gemäß Anspruch 1, dadurch gekennzeichnet, daß man aus die elementaren Multimetalloxidkonstituenten als Bestandteil enthaltenden Ausgangsverbindungen ein inniges Trockengemisch erzeugt und dieses bei Temperaturen von 200 bis 500°C calciniert.

5. Multimetalloxidmassen nach Anspruch 1, die nach einem Verfahren gemäß Anspruch 4 erhalten werden.

6. Verfahren zur gasphasenkatalytischen Oxidation von Acrolein zu Acrylsäure, dadurch gekennzeichnet, daß als Katalysator eine Multimetalloxidmasse gemäß Anspruch 1, 2 oder 5 verwendet wird.

## Claims

1. A multimetal oxide composition which comprises, as basic constituents, the elements Mo, V, W, Cu and Ni in oxidic form, with the proviso that the following molar ratios exist between the various elemental constituents:

Mo:V = 12:1 to 2:1,
Mo:W = 60:1 to 3:1
Mo:Cu = 24:1 to 2:1 and
Cu:Ni = 5:1 to 1:3.

2. A multimetal oxide composition as claimed in claim 1, which conforms to the empirical formula I

$$Mo_{12}V_aW_bCu_cNi_dX_e^1 X_f^2 X_g^3 X_h^4 X_i^5 O_n \tag{I}$$

where

$X^1$ is one or more alkali metals,
$X^2$ is one or more alkaline earth metals,
$X^3$ is chromium, manganese, cerium and/or niobium,
$X^4$ is antimony and/or bismuth,
$X^5$ is silicon, aluminum, titanium and/or zirconium,
a is from 1 to 6,
b is from 0.2 to 4,
c is from 0.5 to 6,
d is from 0.2 to 6,
c:d is from 5:1 to 1:3,
e is from 0 to 2,
f is from 0 to 3,
g is from 0 to 5,
h is from 0 to 40,
i is from 0 to 40 and

n        is a number which is determined by the valency and frequency of the elements in I other than oxygen.

3. The use of a multimetal oxide composition as claimed in claim 1, as a catalyst for the gas-phase catalytic oxidation of acrolein to acrylic acid.

4. A process for the preparation of a multimetal oxide composition as claimed in claim 1, which comprises producing an intimate dry mix of starting compounds which contain the elemental multimetal oxide constituents, and calcining this dry mix at from 200 to 500°C.

5. A multimetal oxide composition as claimed in claim 1, which is obtained by a process as claimed in claim 4.

6. A process for the gas-phase catalytic oxidation of acrolein to acrylic acid, wherein the catalyst used is a multimetal oxide composition as claimed in claim 1, 2 or 5.

**Revendications**

1. Masses d'oxydes de métaux multiples, qui contiennent, à titre de constituants de base, les éléments Mo, V, W, Cu et Mi, sous forme oxydique, qui sont présents dans les rapports molaires suivants entre les divers constituants élémentaires :

   Mo:V = 12:1 à 2:1,
   Mo:W = 60:1 à 3:1,
   Mo:Cu = 24,1 à 2:1 et
   Cu:Ni = 5:1 à 1:3.

2. Masses d'oxydes de métaux multiples suivant la revendication 1, qui possèdent la formule globale I suivante

$$Mo_{12}V_aW_bCu_cNi_dX^1_eX^2_fX^3_gX^4_hX^5_iO_n \qquad (I),$$

dans laquelle les variables ont les significations ci-dessous :

   $X^1$        un ou plusieurs métaux alcalins,
   $X^2$        un ou plusieurs métaux alcalino-terreux,
   $X^3$        chrome, manganèse, cérium et/ou niobium,
   $X^4$        antimoine et/ou bismuth,
   $X^5$        silicium, aluminium, titane et/ou zirconium,
   a        1 à 6,
   b        0,2 à 4,

   c    0,2 à 6,        et c:d =
   d    0,2 à 6,        5:1 à 1:3,

   e        0 à 2,
   f        0 à 3,
   g        0 à 5,
   h        0 à 40,
   i        0 à 40 et
   n        un nombre qui est déterminé par la valence et la fréquence des éléments qui diffèrent de l'oxygène dans I.

3. Utilisation de masses d'oxydes de métaux multiples suivant la revendication 1, à titre de catalyseurs pour l'oxydation catalytique en phase gazeuse de l'acroléine en acide acrylique.

4. Procédé de préparation de masses d'oxydes de métaux multiples suivant la revendication 1, caractérisé en ce que l'on réalise un mélange intime sec à partir des composés de départ contenant, à titre de composant, les constituants des oxydes de métaux multiples élémentaires et que l'on calcine ce mélange intime sec à des températures de 200 à 500°C.

5. Masses d'oxydes de métaux multiples suivant la revendication 1, obtenues suivant le procédé de la revendication 4.

6. Procédé d'oxydation catalytique en phase gazeuse de l'acroléine en acide acrylique, caractérisé en ce que l'on utilise une masse d'oxydes de métaux multiples suivant les revendication 1, 2 ou 5, à titre de catalyseur.